(19) Europäisches Patentamt
European Patent Office
Office européen des brevets

(11) Numéro de publication : **0 101 383**
**B1**

(12) # FASCICULE DE BREVET EUROPÉEN

(45) Date de publication du fascicule du brevet : **12.11.86**

(51) Int. Cl.⁴ : **C 07 J 41/00**, A 61 K 31/57// C07J1/00

(21) Numéro de dépôt : **83401644.6**

(22) Date de dépôt : **10.08.83**

(54) **Dérivés d'amino-14 stéroides, application en thérapeutique, et procédé de préparation.**

(30) Priorité : **12.08.82 FR 8214038**
**17.06.83 FR 8310031**

(43) Date de publication de la demande :
**22.02.84 Bulletin 84/08**

(45) Mention de la délivrance du brevet :
**12.11.86 Bulletin 86/46**

(84) Etats contractants désignés :
**AT BE CH DE FR GB IT LI LU NL SE**

(56) Documents cités :
**EP-A- 0 024 983**
**DD-A- 138 983**
**BULLETIN DE LA SOCIETE CHIMIQUE DE FRANCE, no. 9-10, septembre-octobre 1976, pages 1581-1582, Paris, FR. A. ASTIER et al.: "Alcaloides stéroidiques. CLXXIV. Synthèse d'azides tertiaires. IV.-Azido et amino-14 prégnanes"**
**TETRAHEDRON, vol. 34, no. 10, 1978, pages 1481-1486, Pergamon Press Ltd., Oxford, GB. A. ASTIER et al.: "Alcaloides stéroidiques-CLXXVII: Synthèse d'azides tertiaires-VII. Influence de la configuration des carbones C-5 et C-17 sur l'introduction d'une fonction azide en 14beta à partir de delta-14 steroides"**
**Le dossier contient des informations techniques présentées postérieurement au dépôt de la demande et ne figurant pas dans le présent fascicule.**

(73) Titulaire : **ETABLISSEMENTS NATIVELLE S.A.**
**27, rue de la Procession**
**F-75015 Paris (FR)**

(72) Inventeur : **Jarreau, François-Xavier**
**5 rue Louis Hervé**
**F-78000 Versailles (FR)**
Inventeur : **Koenig, Jean-Jacques**
**31 rue du Panorama**
**F-77672 Vernou-la-Celle s/Seine (FR)**

(74) Mandataire : **L'Helgouaich, Jean**
**OFFICE PICARD 134 Boulevard de Clichy**
**F-75018 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 101 383 B1

**Description**

La présente invention, réalisée dans les laboratoires du CERES — Centre Européen de Recherche Scientifique — concerne de nouveaux amino-14 stéroïdes, leur application en thérapeutique, ainsi qu'un procédé pour leur préparation.

La demande de brevet français 2 464 270 décrit des composés du type amino-14 stéroïdes, et notamment des dérivés hydroxylés de l'amino-14 androstane et de l'amino-14 nor-21 pregnane. On connaît également des alcaloïdes stéroïdiques de la série du pregnane et de l'androstane substitués en position 14 par un groupe amino, et par exemple l'amino-14β pregnanediol-3β,20α est décrit par A. Astier et al., Bull. Soc. Chim. n° 9-10, p. 1581-1582 (1976) ; d'autres amino-14β pregnanes et amino-14β androstanes sont également décrits par A. Astier et al., Tetrahedron vol. 34, p. 1481-1486, (1978). Toutefois aucune propriété pharmacologique ni aucune application thérapeutique de ces dérivés n'a été décrite.

La demande de brevet français 2 494 697 décrit des amino-3 (5α) pregnanediol-17α,20 et amino-3 (5α) nor-19 pregnanol-20, présentés comme possédant des propriétés immunothérapeutiques permettant leur application à titre de médicaments pour le traitement des maladies autoimmunes résultant d'une déficience en certains lymphocytes.

Les travaux réalisés par la demanderesse ont permis de constater de manière surprenante que des amino-14 stéroïdes, et plus particulièrement des dérivés du type pregnanediol-3,20 et pregnanetriol-3,12,20 substitués en position 14 par un groupe amino, possèdent des propriétés inotropes positives.

La présente invention a donc pour objet de nouveaux médicaments à base d'amino-14 stéroïdes, présentant notamment une activité inotrope positive permettant leur application comme médicaments cardiotoniques pour le traitement des insuffisances cardiaques.

L'invention a également pour objet, à titre de produits nouveaux, des amino-14 stéroïdes de la série de l'amino-14 pregnanediol-3,20, l'amino-14 pregnanetriol-3,12,20 et l'amino-14 nor-21 pregnanetriol-3,12,20, ainsi qu'un procédé pour leur préparation.

Les nouveaux médicaments suivant la présente invention contiennent, à titre de principe actif, un amino-14 stéroïde représenté par la formule générale (I) ci-après :

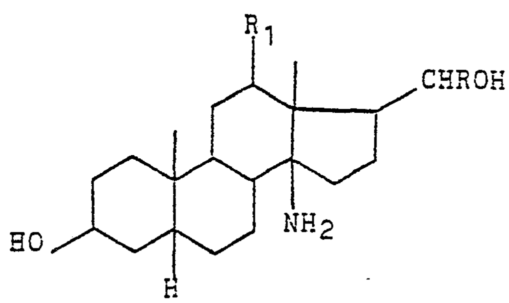

(I)

dans laquelle R représente un atome d'hydrogène ou un groupe alkyle inférieur, comportant 1 à 4 atomes de carbone, et par exemple un groupe méthyle, un groupe éthyle ou un groupe isopropyle, et $R_1$ représente un atome d'hydrogène ou un groupe hydroxyle.

L'invention concerne aussi les sels pharmaceutiquement acceptables des amino-14 stéroïdes de formule générale (I) obtenus par action d'un acide minéral ou organique, suivant les méthodes usuelles de la technique. L'acide utilisé peut être choisi parmi l'acide chlorhydrique, l'acide oxalique, l'acide tartrique, l'acide fumarique, l'acide lactique, l'acide phosphorique, l'acide p-toluènesulfonique, l'acide formique, l'acide bromhydrique, l'acide maléique ou l'acide sulfamique.

L'invention concerne également les nouveaux amino-14 stéroïdes, représentés par la formule générale (I) ci-dessus dans laquelle R représente un atome d'hydrogène ou un groupe alkyle inférieur de 1 à 4 atomes de carbone, et $R_1$ est un atome d'hydrogène ou un groupe hydroxyle, R et $R_1$ n'étant pas simultanément un atome d'hydrogène, ainsi que leurs sels d'acides.

Les amino-14 stéroïdes de formule générale (I) comportent dans leur molécule plusieurs atomes de carbone asymétriques, en particulier les carbones en positions 3, 5, 14, 17 et 21, et peuvent donc exister sous diverses formes stéréoisomères, étant entendu que les nouveaux composés suivant l'invention peuvent être l'un quelconque des ces stéréoisomères dans le cas où $R_1$ est un groupe hydroxyle, tandis que, dans le cas où $R_1$ est un atome d'hydrogène, ils sont constitués par les stéréoisomères pour lesquels, lorsque le groupe OH en position 3 a la configuration β, et lorsque l'atome d'hydrogène en position 17 possède la configuration α, quelle que soit la configuration α ou β de l'atome d'hydrogène en position 5, le groupe OH en position 20 a la configuration β.

L'invention concerne plus particulièrement les amino-14 stéroïdes représentés par la formule générale (I) ci-dessus dans laquelle R est un groupe méthyle. Dans cette formule, le groupe $—NH_2$ en position 14 et l'atome d'hydrogène en position 5 peuvent être en α ou en β, et de préférence en β. De même, le groupe —CHROH en position 17 peut avoir la configuration 17α ou 17β. Le groupe —OH en position 3 et le groupe —OH représenté par $R_1$ ont de préférence la configuration β. Lorsque R n'est pas un atome d'hydrogène,

le groupe —OH en position 20 peut être en configuration α ou β, et de préférence en configuration β dans le cas où $R_1$ est un atome d'hydrogène. L'invention concerne bien entendu ces divers isomères, isolément ou en mélange. Suivant la nomenclature usuelle, dans les exemples ci-après les atomes d'hydrogène en 5 et en 17 occupent la configuration α sauf indication contraire.

Les composés de formule générale (I) peuvent être préparés à partir des dihydroxy-3,14 stéroïdes de formule générale (II) ci-après :

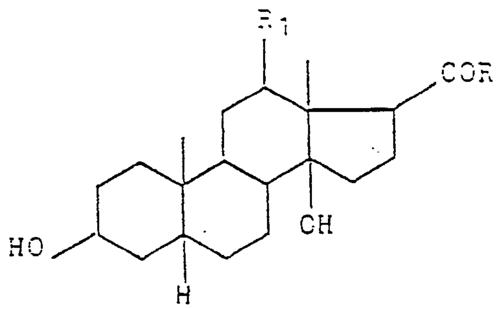

(II)

dans laquelle R représente un atome d'hydrogène ou un groupe alkyle inférieur de 1 à 4 atomes de carbone et $R_1$ représente un atome d'hydrogène ou un groupe hydroxy, par réaction de réduction suivie d'une acétylation pour former les tri-hydroxy-3,14,20 stéroïdes O-acétylés en 3 et 20, et en 12 lorsque $R_1$ est un groupe hydroxy, de formule générale (III) ci-après :

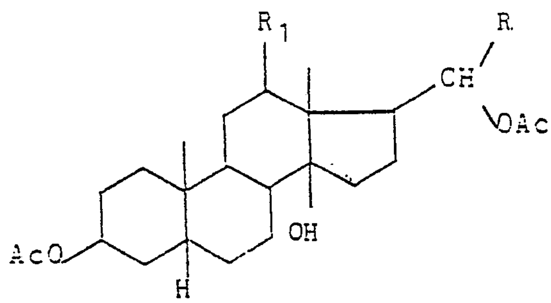

(III)

dans laquelle R est un atome d'hydrogène ou un groupe alkyle inférieur de 1 à 4 atomes de carbone, $R_1$ est un atome d'hydrogène ou un groupe acétyle, et Ac est un groupe acétyle, et en faisant agir un complexe acide azothydrique-trifluorure de bore pour former le dérivé azido-14 correspondant, puis en effectuant une réduction par un hydrure métallique ou par hydrogénation catalytique.

La première étape, consistant à effectuer une réduction suivie d'une acétylation, pour former un trihydroxy stéroïde di-O-acétylé quand $R_1$ est un atome d'hydrogène, ou un tétrahydroxy stéroïde tri-O-acétylé quand $R_1$ est un groupe hydroxy, peut s'effectuer par exemple au moyen d'un hydrure suivant la technique décrite par J. Fried et J. A. Edwards, « Organic Reactions in Steroid Chemistry » Ed. Van Nostrand Reinhold (1972). On peut utiliser par exemple des hydrures mixtes d'aluminium ou de bore, tels que l'hydrure mixte de sodium et de bore ou l'hydrure mixte de lithium et d'aluminium, ou encore d'autres réducteurs tels que l'hydrogène en présence de catalyseurs.

La réaction d'acétylation permettant d'obtenir le stéroïde acétylé de formule générale (III) peut s'effectuer suivant les techniques usuelles, par exemple par action de l'anhydride acétique dans un solvant organique.

Dans la deuxième étape, le dérivé de formule générale (III) est transformé en dérivé azido-14 correspondant par action d'un complexe acide azothydrique-trifluorure de bore en solution dans le benzène. Le remplacement du groupe hydroxyle en position 14 par un groupe azide tertiaire s'effectue avec un bon rendement, les autres groupes hydroxyles en positions 3, 12 le cas échéant, et 20 ayant été préalablement protégés par acétylation.

La troisième étape, consistant à transformer le dérivé azido-14 en dérivé amino-14 correspondant, peut s'effectuer par hydrogénation catalytique suivie d'une élimination des groupes acétyles protecteurs, ou par action d'un réducteur tel qu'un hydrure métallique, et plus particulièrement l'hydrure mixte de lithium et aluminium, pour former l'amino-14 stéroïde de formule (I).

Les cétones de départ, représentées par la formule générale (II) peuvent être préparées en appliquant la méthode décrite par N. Danielli et al. Tetrahedron 22 p. 3189 (1966). Par exemple, lorsque $R_1$ est un groupe hydroxy, on peut traiter un acide diacétoxy-3,12 hydroxy-14 étianique par un organométallique tel que le méthyl-lithium ; il peut être avantageux d'opérer en ajoutant progressivement une solution de méthyl-lithium dans l'éther, à une température inférieure à 10 °C, à une solution d'acide diacétoxy-3,12 hydroxy-14 5β-étianique dans le tétrahydrofuranne en présence d'hydrure de sodium. On peut également faire agir directement un réducteur tel que l'hydrure de lithium aluminium, dans le cas de la préparation

des dérivés où R est l'hydrogène. Le dérivé d'acide étianique utilisé comme produit de départ est décrit dans la littérature (D. Taylor, J. Chem. Soc. 1953, p. 3325). Lorsque $R_1$ est un atome d'hydrogène, on traite de la même façon un acide acétoxy-3 hydroxy-14 étianique par un organométallique.

Les amino-14 stéroïdes de formule générale (I) dans laquelle R et $R_1$ représentent un atome d'hydrogène peuvent également être préparés par le procédé décrit au brevet français 2 464 270. De même, on peut appliquer la méthode de préparation décrite par Astier et al. Bull. Soc. Chim. n° 9-10, p. 1581-1582 (1976) pour obtenir les amino-14 stéroïdes de formule générale (I) où R est un groupe alkyle inférieur et plus précisément un groupe méthyle.

Le procédé conforme à la présente invention permet de préparer dans des conditions satisfaisantes les diverses formes stéréoisomères des dérivés d'amino-14 stéroïdes représentés par la formule générale (I) et notamment les dérivés où les groupes hydroxyles en positions 3 et 12 le cas échéant, et l'atome d'hydrogène en position 5 ont la configuration β, le groupe amino en position 14 possédant de préférence la configuration β, tandis que le groupe hydroxyle en position 20 peut avoir l'une ou l'autre des configurations α et β quand R est un groupe alkyle.

Les exemples décrits ci-après illustrent l'invention sans en limiter la portée. Les structures des produits obtenus ont été vérifiées par spectre infra-rouge, spectre de RNM et spectre de masse.

Exemple 1

a) Préparation du di-O-acétyl-3,20 pregnane-5β triol-3β,14β,20

On dissout 6 g d'oxo-20 pregnane-5β diol-3β,14β obtenu par la méthode de N. Danielli et al. (précité) dans 30 ml de méthanol, puis on ajoute 1,5 g de borohydrure de potassium par fractions, et on laisse réagir pendant 30 min environ. On élimine le méthanol par évaporation, on extrait à l'acétate d'éthyle et on lave. Le résidu est traité par 16,5 ml d'anhydride acétique dans 35 ml de pyridine pendant une nuit. Après hydrolyse, lavage à l'acide citrique et à l'eau, on obtient un résidu (7 g soit un rendement de 95 %) comprenant les deux isomères 20α et 20β que l'on sépare par chromatographie sur colonne de silice.

b) Préparation de l'amino-14β pregnane-5β diol-3β,20α

On dissout 210 mg d'isomère 20α du diacétyltriol obtenu comme indiqué ci-dessus dans 16,5 ml d'acide azothydrique en solution benzénique, et on maintient sous agitation pendant 5 min, puis on ajoute 0,4 ml de réactif éthérate de trifluorure de bore fraîchement distillé. Après 10 min de réaction, le mélange réactionnel est versé sur un mélange de glace pilée et d'ammoniaque.

On extrait au benzène, on lave, on sèche, puis on dissout le résidu de 20 ml de tétrahydrofuranne et on ajoute 54 mg d'hydrure mixte de lithium et d'aluminium. Après chauffage à reflux pendant 1 heure, on hydrolyse et on extrait une fraction neutre de 80 mg et une fraction basique de 77 mg d'aminopregnane-diol (rendement 49 %).

Point de fusion F = 258°C.

Exemple 2

Amino-14β pregnane-5β diol-3β,20β

On dissout 9 g de l'isomère 20β du diacétyltriol préparé comme indiqué dans l'exemple 1a), dans 600 ml d'une solution benzénique d'acide azothydrique, puis on ajoute, après 5 min de réaction, l'éthérate de trifluorure de bore, en effectuant le même traitement que dans l'exemple 1b).

On obtient ainsi 2,3 g d'amino-14β pregnane-5β diol-3β,20β (rendement 32 %). La cristallisation est effectuée dans l'acétate d'éthyle.

Point de fusion F = 196 °C

Spectre IR (Nujol)·$\nu$ = 3 600 à 2 100, 1 585, 1 030 $cm^{-1}$.

Exemple 3

Amino-14α pregnane-5β diol-3β,20α

On procède comme indiqué dans l'exemple 1. Le produit recherché est isolé de son isomère amino-14β pregnane-5β diol-3β,20α par chromatographie sur colonne de silice des eaux-mères de cristallisation du produit principal. La cristallisation est effectuée dans un mélange d'acétate d'éthyle et de méthanol.

Point de fusion F = 188 °C.

Exemple 4

Amino-14α pregnane-5β diol-3β,20β

On procède comme dans l'exemple 2. Le produit recherché est isolé par chromatographie sur colonne de silice des eaux-mères de cristallisation de l'isomère amino-14β pregnane-5β diol-3β,20β.

Le produit est cristallisé dans l'isopropanol.

Point de fusion F = 216 °C.

Exemple 5

Amino-14β pregnane-5β,17βH diol-3β,20α

On réduit 0,5 g d'oxo-20 pregnane-5β,17βH diol-3β,14β par 120 mg de borohydrure de potassium dans 3 ml de méthanol. Après réaction, on effectue une acétylation par l'anhydride acétique dans la pyridine suivant la technique usuelle, pour obtenir avec un rendement de 95 % le di-O-acétyl pregnane-5β,17βH triol-3β,14β, 20α.

On dissout 0,4 g du diacétyltriol obtenu comme indiqué ci-dessus dans 30 ml d'une solution benzénique d'acide azothydrique, puis on ajoute 0,6 ml d'éthérate de trifluorure de bore. Après traitement comme dans l'exemple 1, on fait agir 0,1 g d'hydrure mixte de lithium et d'aluminium sur le résidu dans 10 ml de tétrahydrofuranne. Après hydrolyse, extraction et cristallisation dans l'acétate d'éthyle on obtient 0,25 g (rendement 80 %) d'amino-14β pregnane-5β,17βH diol-3β,20α.

Point de fusion F = 200-201 °C.

Exemple 6

Amino-14β pregnane-5α diol-3β,20α

On procède comme dans l'exemple 1 avec l'isomère 5α en laissant agir l'éthérate de trifluorure de bore environ 5 heures.

Après traitement et réduction par l'hydrure mixte de lithium et d'aluminium comme dans l'exemple 1, le résidu est repris dans l'acétate d'éthyle puis lavé par une solution d'acide chlorhydrique à 10 %. L'acétate d'éthyle est éliminé par évaporation et les phases acides sont neutralisées par du carbonate de sodium et extraites au chloroforme. Après filtration, le filtrat est séché et fournit après cristallisation dans l'acétate d'éthyle, l'amino-14β pregnane-5α diol-3β,20α avec un rendement supérieur à 50 %.

Point de fusion F = 246 °C.

Exemple 7

Amino-14β pregnane-5α diol-3β,20β

On procède comme dans l'exemple 1a) à partir de l'isomère 5α du pregnanediol de départ, et on isole l'isomère 20β du diacétyltriol obtenu.

On applique ensuite le même traitement que dans l'exemple 1b), et on obtient ainsi l'amino-14β pregnane-5α diol-3β,20β que l'on recristallise dans l'éther diisopropylique.

Point de fusion F = 213 °C.

Exemple 8

Amino-14β nor-21 pregnane-5β diol-3β,20.

Ce composé est préparé comme indiqué dans l'exemple 6 du brevet français 2 464 270 au nom de la demanderesse.

Exemple 9

Amino-14β pregnane-5β triol-3β,12β,20β

On ajoute 1,1 g d'hydrure de lithium aluminium, en maintenant la température à 0 °C, à une solution de 130 ml de tétrahydrofuranne contenant 8 g d'un mélange d'oxo-20 pregnane-5β triol-3β,12β,14β et de dérivés O-acétylés correspondants, obtenu en faisant agir du méthyl lithium, en solution dans l'éther, sur l'acide diacétoxy-3β,12β hydroxy-14β 5β-étianique dans le tétrahydrofuranne en présence d'hydrure de sodium, à une température inférieure à 10 °C environ, sous atmosphère d'azote.

On ajoute à froid de l'acétate d'éthyle, puis un mélange de tétrahydrofuranne et d'eau. Après filtration et évaporation du solvant, on recueille 7,6 g de pregnane-5β tétrol-3β,12β,14β,20ξ sous forme de poudre incolore.

Le produit ci-dessus est dissous dans 120 ml de chlorure de méthylène et on ajoute à la solution 9,2 ml d'anhydride acétique et 1,2 g de diméthylamino-4 pyridine. On maintient le mélange réactionnel sous agitation pendant quelques heures puis on lave par une solution de carbonate de sodium et on extrait la

phase aqueuse avec du chlorure de méthylène. Après séchage, on obtient 9,3 g de tri-O-acétyl-3,12,20 pregnane-5β tétrol-3β,12β,14β,20ξ.

Les deux isomères 20α et 20β sont séparés par chromatographie sur silice Merck H60 en utilisant comme éluant un mélange de chlorure de méthylène et d'acétone (96,5/3,5).

Le tri-O-acétyl-3,12,20 pregnane-5β tétrol-3β,12β,14β,20β est recristallisé dans l'éther (point de fusion F = 216 °C). On en dissout 5,7 g dans 330 ml d'une solution benzénique d'acide azothydrique environ 10M, puis on ajoute 7,3 ml d'éthérate de trifluorure de bore fraîchement distillé et on maintient sous agitation pendant environ 25 min.

Après alcalinisation par un mélange d'ammoniaque et de glace pilée, extraction au benzène, lavage à l'eau, séchage sur sulfate de sodium et évaporation, on obtient 5,8 g d'un résidu que l'on dissout dans 300 ml d'éthanol. On effectue une hydrogénation à température ambiante sous atmosphère d'hydrogène pendant 48 heures en présence de 2,9 g de palladium sur carbonate de calcium (5 %).

Le catalyseur est éliminé par filtration et le filtrat est évaporé à sec pour fournir 5,5 g d'une mousse incolore que l'on dissout dans le toluène.

On lave plusieurs fois avec une solution d'acide sulfamique à 5 %. Le précipité formé est filtré et purifié pour fournir 0,5 g de tri-O-acétyl-3,12,20 amino-14β pregnane-5β triol-3β,12β,20β. Les phases aqueuses acides sont alcalinisées au carbonate de sodium et extraites au chlorure de méthylène pour procurer 0,75 g du même produit.

Point de fusion F = 211 °C (éther isopropylique).

On saponifie 0,2 g de tri-O-acétyl-3,12,20 amino-14β pregnane-5β triol-3β,12β,20β, obtenu comme indiqué ci-dessus dans 150 ml de soude méthanolique 1N en agitant la suspension pendant 2 heures à température ambiante, puis en chauffant à reflux pendant 30 min.

Après évaporation du solvant, le résidu est extrait par le chloroforme. On obtient ainsi 0,15 g d'amino-14β pregnane-5β triol-3β,12β,20β, que l'on peut faire recristalliser dans le mélange acétate d'éthyle-méthanol.

Point de fusion F = 238 °C.

Spectre IR (Nujol) $\nu$ = 3 400, 3 330, 3 270, 3 180, 3 100, 2 660, 1 615, 1 600 $cm^{-1}$.

Les amino-14 stéroïdes conformes à l'invention, représentés par la formule générale (I) ci-dessus, possèdent d'intéressantes propriétés pharmacologiques, et plus particulièrement, ils possèdent une activité inotrope positive.

Ces propriétés, illustrées ci-après, montrent que les amino-14 stéroïdes de formule générale (I) ainsi que leurs sels pharmaceutiquement acceptables, peuvent être utilisés en thérapeutique humaine et vétérinaire comme médicaments destinés au traitement de l'insuffisance cardiaque.

Les expérimentations toxicologiques effectuées ont montré que la toxicité aiguë des amino-14 stéroïdes suivant l'invention est relativement faible, et en particulier nettement plus faible que celle des composés digitaliques usuels tels que la digitoxine et la digoxine.

La mise en évidence de l'effet inotrope *in vitro* a été faite sur le cœur isolé de cobaye selon la méthode de Langendorff et sur l'oreillette isolée de cobaye. Les résultats obtenus démontrent la possibilité d'application pour le traitement de l'insuffisance cardiaque.

A titre d'exemple, dans le cas de l'amino-14β pregnane-5β triol-3β,12β,20β, le test du cœur isolé perfusé selon Langendorff fait apparaître une augmentation du débit cardiaque de 30 à 40 % environ pour des doses de 1 à 30 μg/ml. Dans le test sur l'oreillette isolée de cobaye, pour une concentration de $10^{-5}$ g/l on constate une augmentation de la force de contraction de 150 % environ par rapport à la valeur contrôle. Ces résultats sont confirmés en faisant varier les conditions expérimentales, après administration de propranolol, apportant la preuve de l'action myocardique directe du composé et en éliminant une médiation adrénergique.

Des résultats comparables sont obtenus avec l'amino-14β nor-21 pregnane-5β diol-3β,20.

Les dérivés de formule générale (I) et leurs sels pharmaceutiquement acceptables peuvent être administrés sous les formes usuelles, le principe actif étant dilué dans un support pharmaceutiquement acceptable convenablement choisi, par exemple sous forme de comprimé, gélule, dragée, suppositoire, soluté injectable ou sirop.

A titre d'exemple, les comprimés peuvent être préparés en mélangeant le dérivé de formule générale (I) ou un de ses sels, avec un ou plusieurs diluants solides tels que le lactose, le mannitol, l'amidon, la polyvinylpyrrolidone, le stéarate de magnésium ou le talc. Le cas échéant, les comprimés peuvent comporter plusieurs couches superposées autour d'un noyau, suivant les techniques usuelles, pour assurer une libération progressive ou un effet retardé du principe actif. L'enrobage peut par exemple être constitué d'une ou plusieurs couches d'acétate de polyvinyle, de carboxyméthylcellulose ou d'acétophtalate de cellulose.

On peut également administrer le dérivé suivant l'invention sous forme d'un sirop ou d'un soluté buvable obtenu en dissolvant le dérivé de formule (I) ou un de ses sels pharmaceutiquement acceptables, dans de l'eau ou du glycérol, par exemple, et en ajoutant le cas échéant un additif usuel tel qu'un édulcorant et un antioxydant.

Des solutions injectables peuvent être préparées suivant les techniques bien connues et sont constituées par exemple par un soluté contenant un dérivé de formule (I) ou un de ses sels pharmaceutiquement acceptables, dissous dans de l'eau bidistillée, une solution hydroalcoolique ou du propylèneglycol, ou un mélange de ces solvants. Le cas échéant, un additif approprié tel qu'un

conservateur peut être ajouté.

Les doses administrées sont déterminées par le médecin en fonction du mode d'administration choisi, le niveau de l'affection traitée ou la durée du traitement. Par exemple, dans le cas de l'administration par voie orale chez l'homme, les doses peuvent être comprises entre 0,005 et 5 mg/kg.

**Revendications** (pour les Etats contractants : BE, CH, DE, FR, GB, IT, LI, LU, NL, SE)

1. Médicaments caractérisés en ce qu'ils contiennent un amino-14 stéroïde représenté par la formule générale (I) :

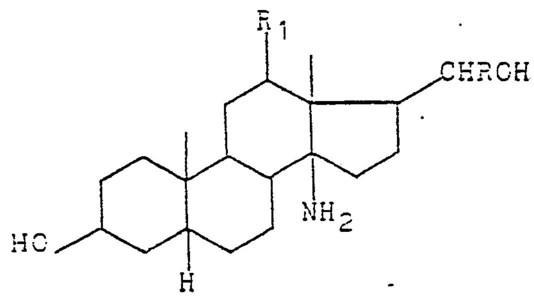

(I)

dans laquelle R représente un atome d'hydrogène ou un groupe alkyle inférieur comportant 1 à 4 atomes de carbone, et $R_1$ représente un atome d'hydrogène ou un groupe hydroxyle, ainsi que leurs sels pharmaceutiquement acceptables.

2. Médicaments selon la revendication 1, caractérisés en ce que R est un atome d'hydrogène ou un groupe méthyle.

3. Médicaments selon la revendication 2, caractérisés en ce que le groupe $-NH_2$ en position 14 possède la configuration α.

4. Médicaments selon la revendication 2, caractérisés en ce que le groupe $-NH_2$ en position 14 possède la configuration β.

5. Amino-14 stéroïdes, caractérisés en ce qu'ils sont représentés par la formule générale (I) :

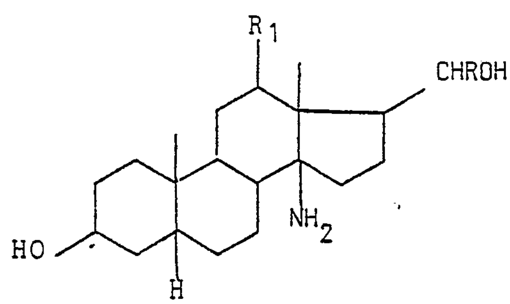

(I)

dans laquelle R est un atome d'hydrogène ou un groupe alkyle inférieur comportant 1 à 4 atomes de carbone, et $R_1$ est un atome d'hydrogène ou un groupe hydroxyle, R et $R_1$ n'étant pas simultanément un atome d'hydrogène, étant entendu que, quand $R_1$ est un atome d'hydrogène, le groupe —OH en position 20 a la configuration β lorsque le groupe —OH en position 3 a la configuration β, et lorsque l'atome d'hydrogène en position 17 possède la configuration α.

6. Amino-14 stéroïdes selon la revendication 5, caractérisés en ce que R est un groupe méthyle.

7. Procédé de préparation d'amino-14 stéroïdes de formule générale (I) :

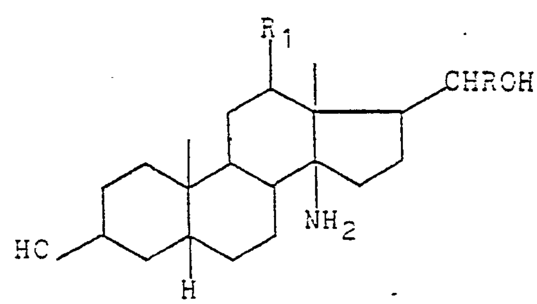

(I)

dans laquelle R est un atome d'hydrogène ou un groupe alkyle inférieur de 1 à 4 atomes de carbone, et $R_1$ représente un atome d'hydrogène ou un groupe hydroxyle, caractérisé en ce qu'on effectue une

réduction puis une acétylation des trihydroxy-3,12,14 stéroïdes de formule (II)

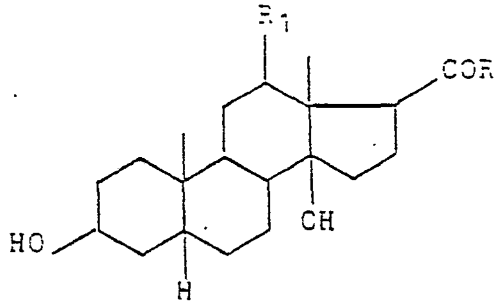

(II)

dans laquelle R a la même définition que ci-dessus, pour former les tétra-hydroxy-3,12,14,20 stéroïdes O-acétylés de formule (III) :

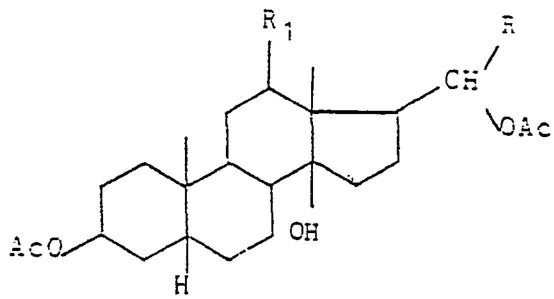

(III)

dans laquelle R a la même signification que ci-dessus, $R_1$ est un atome d'hydrogène ou un groupe acétyle et Ac est un groupe acétyle, sur lesquels on fait agir un complexe acide azothydrique-trifluorure de bore pour former le dérivé azido-14 correspondant, puis on effectue une réduction par un hydrure métallique ou par hydrogénation catalytique.

**Revendication** (pour l'Etat contractant AT)

Procédé de préparation d'amino-14 stéroïdes de formule générale (I) :

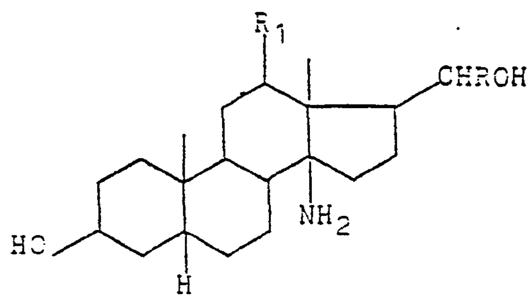

(I)

dans laquelle R est un atome d'hydrogène ou un groupe alkyle inférieur de 1 à 4 atomes de carbone, et $R_1$ représente un atome d'hydrogène ou un groupe hydroxyle, caractérisé en ce qu'on effectue une réduction puis une acétylation des trihydroxy-3,12,14 stéroïdes de formule (II)

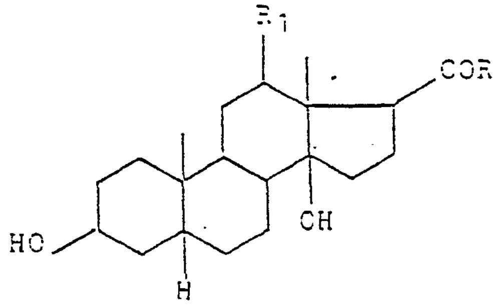

(II)

dans laquelle R a la même définition que ci-dessus, pour former les tétra-hydroxy-3,12,14,20 stéroïdes O-acétylés de formule (III) :

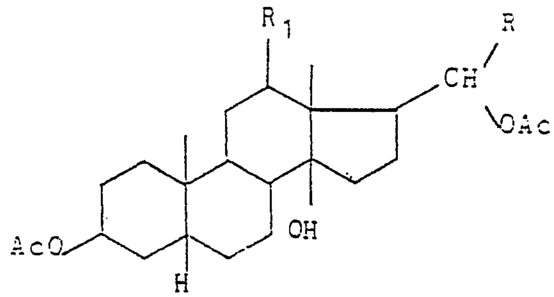

(III)

dans laquelle R a la même signification que ci-dessus, $R_1$ est un atome d'hydrogène ou un groupe acétyle et Ac est un groupe acétyle, sur lesquels on fait agir un complexe acide azothydrique-trifluorure de bore pour former le dérivé azido-14 correspondant, puis on effectue une réduction par un hydrure métallique ou par hydrogénation catalytique.

**Claims** (for the Contracting States : BE, CH, DE, FR, GB, IT, LI, LU, NL, SE)

1. Medicaments characterized in that they contain a 14-aminosteroid represented by the general formula (I) :

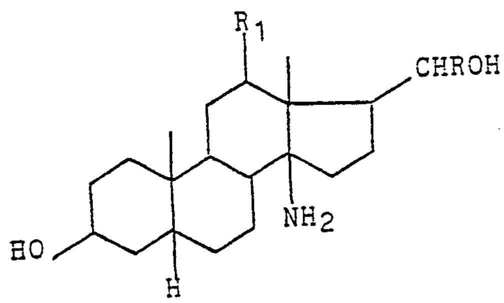

(I)

in which R represents a hydrogen atom or a lower alkyl group containing 1 to 4 carbon atoms, and $R_1$ represents a hydrogen atom or a hydroxyl group, or the pharmaceutically acceptable salts thereof.

2. Medicaments as claimed in claim 1, characterized in that R is a hydrogen atom or a methyl group.

3. Medicaments as claimed in claim 2, characterized in that the $-NH_2$ group in the 14-position possesses the α configuration.

4. Medicaments as claimed in claim 2, characterized in that the $-NH_2$ group in the 14-position possesses the β configuration.

5. 14-aminosteroids characterized in that they are represented by the general formula (I)

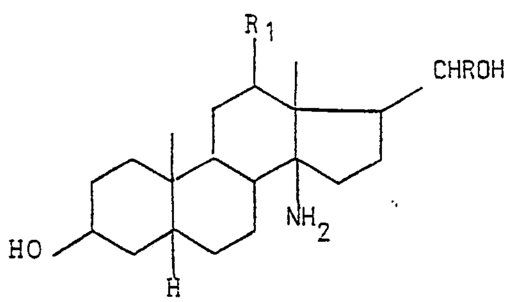

(I)

in which R is a hydrogen atom or a lower alkyl group containing 1 to 4 carbon atoms, and $R_1$ is a hydrogen atom or a hydroxyl group, R and $R_1$ not being a hydrogen atom simultaneously, it being understood that when $R_1$ is a hydrogen atom, the —OH group in the 20-position has the β configuration when the —OH group in the 3-position has the β configuration and when the hydrogen atom in the 17-position possesses the α configuration.

6. 14-aminosteroids as claimed in claim 5, characterized in that R is a methyl group.

7. A process for the preparation of 14-aminosteroids of the general formula (I) :

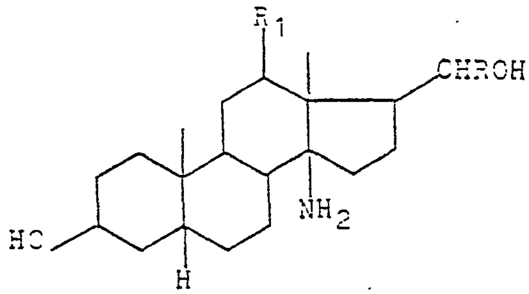

(I)

in which R is a hydrogen atom or a lower alkyl group having 1 to 4 carbon atoms, and $R_1$ represents a hydrogen atom or a hydroxyl group, characterized in that it comprises reducing and then acetylating a 3,12,14-tri-hydroxysteroid of the formula (II) :

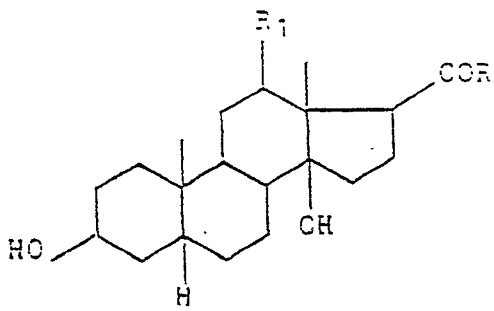

(II)

in which R has the same definition as above, to form O-acetylated 3,12,14,20-tetrahydroxysteroids of the formula (III) :

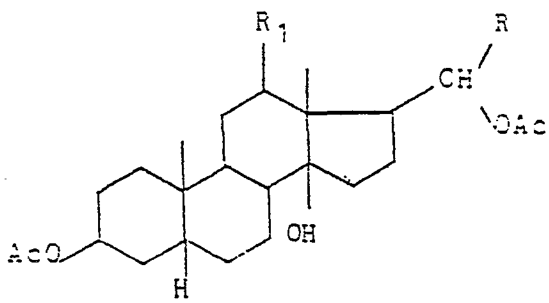

(III)

in which R has the same meaning as above, $R_1$ is a hydrogen atom or an acetyl group and Ac is an acetyl group, reacting a hydrazoic acid/boron trifluoride complex with the compounds of the formula (III) to form the corresponding 14-azido derivative, and then reducing this with a metal hydride or by catalytic hydrogenation.

**Claim** (for the Contracting State AT)

A process for the preparation of 14-aminosteroids of the general formula (I) :

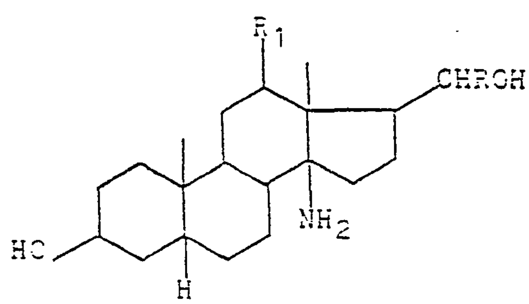

(I)

in which R is a hydrogen atom or a lower alkyl group having 1 to 4 carbon atoms, and $R_1$ represents a hydrogen atom or a hydroxyl group, characterized in that it comprises reducing and then acetylating a 3,12,14-tri-hydroxysteroid of the formula (II) :

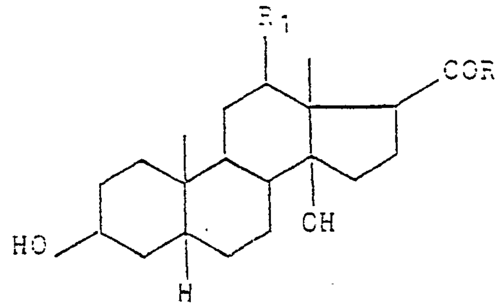

(II)

in which R has the same definition as above, to form O-acetylated 3,12,14,20-tetrahydroxysteroids of the formula (III) :

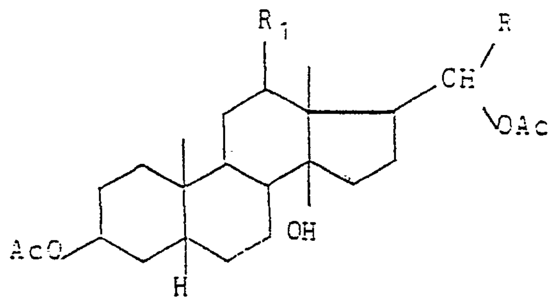

(III)

in which R has the same meaning as above, $R_1$ is a hydrogen atom or an acetyl group and Ac is an acetyl group, reacting a hydrazoic acid/boron trifluoride complex with the compounds of the formula (III) to form the corresponding 14-azido derivative, and then reducing this with a metal hydride or by catalytic hydrogenation.

**Patentansprüche** (für die Vertragsstaaten : BE, CH, DE, FR, GB, IT, LI, LU, NL, SE)

1. Heilmittel, dadurch gekennzeichnet, daß sie ein 14-Aminosteroid der allgemeinen Formel (I)

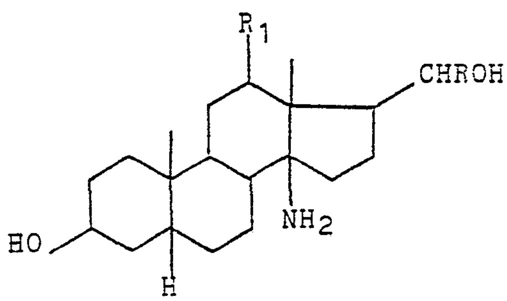

(I)

in welcher R ein Wasserstoffatom oder eine niedere Alkylgruppe mit 1 bis 4 Kohlenstoffatomen und $R_1$ ein Wasserstoffatom oder eine Hydroxygruppe bedeutet, und ihre pharmazeutisch annehmbaren Salze enthalten.

2. Heilmittel nach Anspruch 1, dadurch gekennzeichnet, daß R ein Wasserstoffatom oder eine Methylgruppe bedeutet.

3. Heilmittel nach Anspruch 2, dadurch gekennzeichnet, daß die in 14-Stellung befindliche $NH_2$-Gruppe α-Konfiguration besitzt.

4. Heilmittel nach Anspruch 2, dadurch gekennzeichnet, daß die in 14-Stellung befindliche $NH_2$-Gruppe β-Konfiguration besitzt.

5. 14-Aminosteroide, dadurch gekennzeichnet, daß sie der allgemeinen Formel (I)

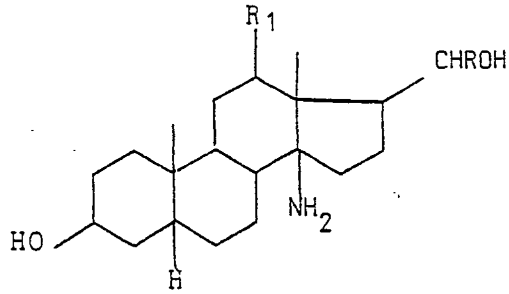

(I)

entsprechen, in welcher R ein Wasserstoffatom oder eine niedere Alkylgruppe mit 1 bis 4 Kohlenstoffatomen und $R_1$ ein Wasserstoffatom oder eine Hydroxygruppe bedeutet, wobei R und $R_1$ nicht gleichzeitig für ein Wasserstoffatom stehen können und, falls $R_1$ ein Wasserstoffatom bedeutet, die in 20-Stellung befindliche —OH-Gruppe β-Konfiguration besitzt, wenn die in 3-Stellung befindliche —OH-Gruppe β-Konfiguration und das in 17-Stellung befindliche Wasserstoffatom α-Konfiguration besitzt.

6. 14-Aminosteroide nach Anspruch 5, dadurch gekennzeichnet, daß R eine Methylgruppe ist.

7. Verfahren zum Herstellen von 14-Aminosteroiden der allgemeinen Formel (I)

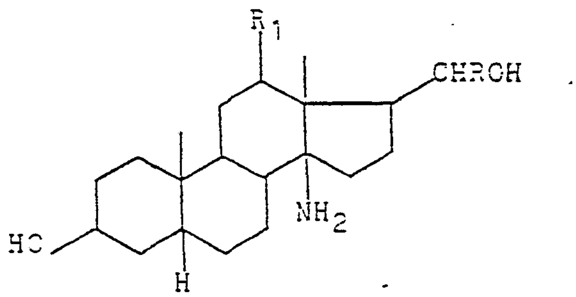

(I)

in welcher R ein Wasserstoffatom oder eine niedere Alkylgruppe mit 1 bis 4 Kohlenstoffatomen und $R_1$ ein Wasserstoffatom oder eine Hydroxygruppe bedeutet, dadurch gekennzeichnet, daß 3,12,14-Trihydroxysteroide der Formel (II)

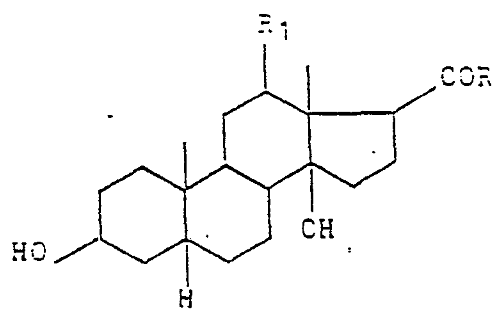

(II)

in welcher R die oben angegebene Bedeutung besitzt, durch Reduktion und anschließendes Acetylieren in O-acetylierte 3,12,14,20-Tetrahydroxy-steroide der Formel (III)

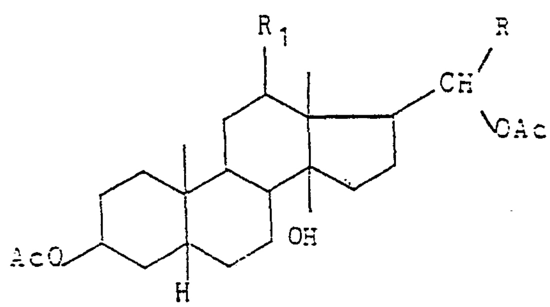

(III)

in welcher R die oben angegebene Bedeutung besitzt, $R_1$ ein Wasserstoffatom oder eine Acetylgruppe bedeutet und Ac eine Acetylgruppe darstellt, übergeführt werden, auf welche zwecks Herstellung des entsprechenden 14-Azido-derivats ein Stickstoffwasserstoffsäure-Bortrifluorid-Komplex einwirken gelassen wird, worauf eine Reduktion mittels eines Metallhydrids oder durch katalytisches Hydrieren vorgenommen wird.

**Patentanspruch** (für den Vertragsstaat AT)

Verfahren zum Herstellen von 14-Aminosteroiden der allgemeinen Formel (I)

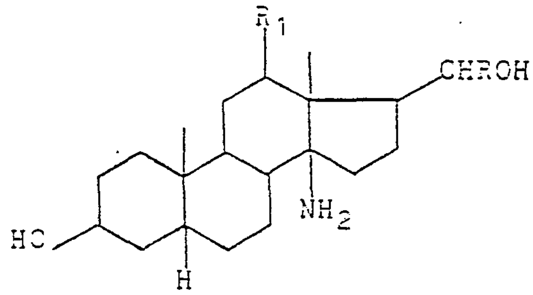

(I)

in welcher R ein Wasserstoffatom oder eine niedere Alkylgruppe mit 1 bis 4 Kohlenstoffatomen und $R_1$ ein Wasserstoffatom oder eine Hydroxygruppe bedeutet, dadurch gekennzeichnet, daß 3,12,14-Trihydroxy-steroide der Formel (II)

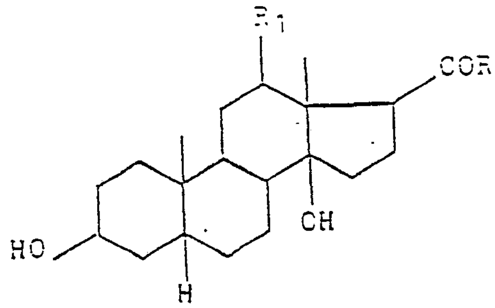

(II)

in welcher R die oben angegebene Bedeutung besitzt, durch Reduktion and anschließendes Acetylieren in O-acetylierte 3,12,14,20-Tetrahydroxy-steroide der Formel (III)

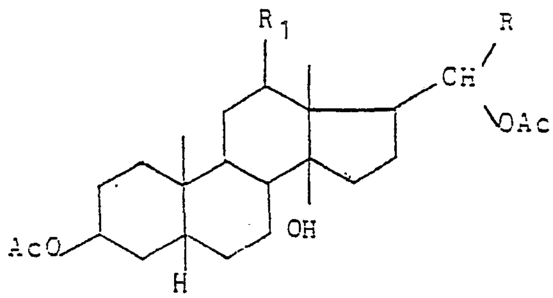

(III)

in welcher R die oben angegebene Bedeutung besitzt, $R_1$ ein Wasserstoffatom oder eine Acetylgruppe bedeutet und Ac eine Acetylgruppe darstellt, übergeführt werden, auf welche zwecks Herstellung des entsprechenden 14-Azido-derivats ein Stickstoffwasserstoffsäure-Bortrifluorid-Komplex einwirken gelassen wird, worauf eine Reduktion mittels eines Metallhydrids oder durch katalytisches Hydrieren vorgenommen wird.